# EUROPEAN PATENT SPECIFICATION

(11) **EP 2 281 836 B1**
(45) Date of publication and mention of the grant of the patent: **14.03.2012**
(21) Application number: 10171705.6
(22) Date of filing: 03.08.2010
(51) Int. Cl.: C07K 14/415, C12N 15/62, A61K 39/36

(54) **Hybrid proteins from Parietaria judaica major allergens and uses thereof**
Hybridproteine von Parietaria major Allergenen und deren Verwendungen
Protéines hybrides d'allergènes de Parietaria major et leurs utilisations

(30) Priority: 04.08.2009 IT MI20091411
(43) Date of publication of application: 09.02.2011
(73) Proprietor: LOFARMA S.p.A., 20143 Milano (IT)
(72) Inventor: Mistrello, Giovanni, 20143 Milano (IT); Zanotta, Stefania, 20143 Milano (IT); Roncarolo, Daniela, 20143 Milano (IT); Falagiani, Paolo, 20143 Milano (IT)
(74) Representative: Banfi, Paolo

(56) References cited:
- EP-A1- 1 712 560
- WO-A1-2004/104047
- WO-A1-2005/085278
- WO-A1-2007/116316
- WO-A2-02/22674
- ANDRES ASTURIAS JUAN: "Recombinant hypoallergens for immunotherapy of Parietaria judaica pollen allergy" FRONTIERS IN BIOSCIENCE, vol. 14, January 2009 (2009-01), pages 4606-4617, XP002563510 ISSN: 1093-9946

## Description

The present invention provides dimeric hypoallergenic proteins of *Parietaria judaica,* nucleic acid molecules encoding for them, pharmaceutical compositions containing the same, and their use in the immunotherapy of allergic diseases caused by pollen of plants from *Parietaria judaica* species.

### BACKGROUND OF THE INVENTION

Allergies are caused by an abnormality in the immune system that reacts by producing IgE antibodies to proteins which, per se, are completely harmless and mainly contained in pollens, acari, epithelia, and some foods.

Recent estimates show that more than 25% of people in industrialized nations are affected by this disease, that, persisting in time, may induce worsening of symptoms (e.g., onset of asthma), and sensitization to other allergens, thus making the choice of the most appropriate therapy more complicated (1).

Hyposensitizing specific immunotherapy (SIT), unlike pharmacological therapy, is the only form of etiological treatment of allergic diseases that is able to positively affect some immunological parameters that are the basis of the disease.

SIT consists of administration of increasing doses of standardized extracts (vaccines) obtained from the same substance causing the disease (2).

In this way, a sort of "immunological tolerance" towards such substance is gradually induced in patients. This immunological tolerance is associated to a reduction or even disappearance of allergic symptoms.

The risk for eliciting side effects that are even severe in nature (3), also if it is considerably reduced by the use of slow-release vaccines or vaccines that are administered by alternative routes to injections, has limited SIT applicability in the therapy of allergic diseases. Moreover, as SIT is carried out by administering a mix of allergenic and non-allergenic proteins of natural origin without taking account of patient's sensitization profile, new IgE reactivities to initially harmful allergens that are present in the extract can arise.

An element related to the benefit caused by SIT is induction of IgG antibodies specific for the sensitizing allergen. These (protective) antibodies may inhibit IgE binding to antigen, thus influencing the tridimensional structure of this molecule (4, 5). The development of vaccines made of recombinant proteins having less allergenicity but an unaltered immunogenic capacity might result in a further improvement in the field of allergic disease therapy.

During the last 15 years, considerable advances have been made in the field of allergen characterization thanks to the application of recombinant DNA-based technologies. cDNAs coding for main allergens are available for allergenicity studies and the development of diagnostic tests, and enable innovative SITs based on the use of purified recombinant proteins and their genetically modified variants that are characterized by reduced allergenic activity (6).

Parietaria pollen is one of the most important causes of allergy in the Mediterranean area. In particular, at least 50% of all allergic subjects in Southern Italy show a positive response to skin prick test (SPT) for Parietaria pollen (7).

The two main allergens of *Parietaria judaica* pollen, i.e. Par j 1 (whose nucleotide sequence is identified by GenBank Acc. No. X95867), and Par j 2 (AC X95865), are proteins with molecular weights of 14.4 and 11.3 kD, respectively, and share sequence and functional homology (8, 9). The percentage of sequence identity is around 45%, and is more prominent in the N-terminal region of the proteins, where the most important IgE epitope is located. This epitope is responsible for much of cross-reactivity between the two molecules (10, 11). These two independent allergens exhibit common features, and belong to a family of proteins of plant origin that are called ns-LTPs (non-specific Lipid Transfer Proteins) and characterized by a highly conserved tridimensional structure (12). These proteins have the function to transport lipid molecules through the membrane.

Numerous studies have been performed to identify or alter IgE epitopes of major Parietaria allergens.

By changing some cysteines that are relevant for maintaining the tridimensional structure in both allergens, tridimensional mutants with reduced IgE binding and decreased allergenicity to humans have been obtained by site-specific mutagenesis (13).

The use of overlapping synthetic peptides corresponding to the entire Par j 1 and Par j 2 sequences allowed the detection of linear epitopes that are capable of binding IgEs of allergic patients to Parietaria. Positioning of these sequences on the tridimensional model of the two allergens allowed the localization of three regions with strong IgE reactivity and a high homology degree in both molecules (11).

WO02/22674 (14) discloses a hypoallergenic variant of major allergen Par j 2 (corresponding to SEQ ID NO:10 shown below), in which the change in 8 lysines distributed throughout the protein causes a strong decrease in binding capability to specific IgEs.

EP1712560 (15) identifies the amino acids involved in binding reactions between specific IgEs and Par j 1. The different combinations of mutagenized residues give rise to molecules with different degrees of allergenicity, and show the presence of multiple epitopes that are variably recognized by patients allergic to *Parietaria judaica.*

The possibility to use engineered multimeric molecules made of different allergens from the same organism for hyposensitizing specific therapy has long been considered fascinating.

Allergy to Parietaria pollen is an optimal study model as this allergy is mainly caused by the two allergenic components, Par j 1 and Par j 2, a mix of which is capable of inhibiting the binding between specific IgEs and *Parietaria judaica* pollen up to 95% (16), thus proving that other allergens in the extract are less clinically important. It is known that Par j 1 and Par j 2 share numerous IgE epitopes, and that Par j 2 can be used as a marker for the diagnosis of Parietaria allergy.

The association of Par j 1 and Par j 2 in a single hybrid molecule would represent the whole repertoire of T epitopes, and might induce a strong protective IgG response.

W02004/104047 (17) proposes a multimer comprising at least a first amino acid sequence corresponding to one of Parietaria Par j 1 or Par j 2 major allergens, a second sequence coding for Par j 1 or Par j 2, and preferably a third sequence from one of the two Parietaria major allergens. The subject of the invention are also multimeric molecules in which major allergens are mutated in the region between amino acids 1 to 30, preferably at positions Lys 23, Glu 24, and Lys 27. In the Results section, the ability to induce histamine release from wt or mutagenized Parj2-Parj 1 dimer is analyzed compared to an equimolar mix of the single recombinant Par j 1 and Par j 2 allergens. It is reported (Fig. 6, page 12) that wt dimer (PjED clone) induces less histamine release in two out of the three tested patients (panels G and H), while mutagenized dimer (PjEDmut clone) is equal to wt dimer in two out of the three patients, and is less effective in one out of the three (panel F), thus showing that the mutations inserted in PjEDmut clone at positions Lys 23, Glu 24 and Lys 27 are not crucial for the specific binding to IgEs from all allergic patients.

In a paper dated 2007 (18), Bonura et al. tested the Par j 2-Par j 1 hybrid by comparing it with its derivative, rPjEDcys, which had been mutagenized in both allergens at cysteines that are important for maintaining the tridimensional structure. The mutated rPjEDcys hybrid has reduced IgE reactivity, as demonstrated in ELISA inhibition and SPT experiments, and by induction of histamine release from basophiles. Moreover, the mutagenized variant maintains its T epitopes unchanged because, when injected into mice, it is able of inducing an IgG production capable of recognizing the single Par j 1 and Par j 2 allergens.

In the same year, Gonzalez-Rioja et al. (16) tested three different hybrid molecules made of the two Parietaria major allergens, Par j 1 and Par j 2, in wild-type (Q1) form, or in the form of mutants in which only the 29-52 fragment containing the 4 cysteines involved in the four disulphide bonds (Q2), and this fragment plus a further deletion of 8 residues (amino acids 72-79 of Par j 1, and 73-80 of Par j 2, Q3) corresponding to two putative linear IgE epitopes, were deleted in both proteins. The data show that Q1 hybrid has lower IgE reactivity compared to the mix of the two single allergens, and a further decrease is observed with Q2 and Q3. The ability to stimulate T lymphocytes remains unaltered for Q1 and Q2 hybrids compared to the mix of the single proteins, while it significantly decreases in the case of Q3, indicating that deleting these sequences removes important T epitopes for a protective antibody response, see also WO2007/116316.

In the two above cited papers, decrease in allergenicity of molecules is related to mutation of some cysteines known to be crucial for maintaining their tridimensional structure, and hence conformational B epitopes.

The results obtained with major allergens mutagenized at amino acids that are not directly involved in 3D structure stabilization (17) proved to be scarcely encouraging because the oligomeric mutant showed no significant decrease in allergenicity, not only with respect to the mix of the single allergenic wt components but also compared to the wt dimeric molecule. These molecules do not seem to be effective candidates for use as a unique hypoallergenic ingredient in the therapy of *Parietaria judaica* pollen allergy.

### DISCLOSURE OF THE INVENTION

It has now been found that binding of Par j 1 and Par j 2 allergens to IgEs can be reduced by using them in the form of a hybrid protein made of Par j 1 allergen sequence linked to Par j 2 sequence, in which both sequences are mutated through substitution of amino acid residues.

According to a first embodiment, the invention provides a hybrid protein consisting of mutated Par j 1 and Par j 2 sequences linked to each other in any order either directly or by interposition of a linker, wherein:
a) in Par j 1 sequence - which is either SEQ ID NO: or a sequence of a natural protein that is identical to SEQ ID NO: by at least 95%, preferably at least 98% - the Lys residues at positions 23, 27, 41, and 66 of SEQ ID NO:1, or at corresponding positions of said natural sequence, are replaced by neutral, polar, or acidic amino acids;
b) in Par j 2 sequence - which is either SEQ ID NO:2 or a sequence of a natural protein that is identical to SEQ ID NO:2 by at least 95%, preferably at least 98% - the Lys residues at positions 19, 23, 27, 35, 41, 46, 73, and 78 of SEQ ID NO:2, or at corresponding positions of said natural sequence, are replaced by neutral, polar, or acid amino acids;
c) said Par j 1 and Par j 2 sequences have a head-to-tail orientation;
d) if present, said linker consists of an amino acid chain containing 1 to 8 amino acids.

The above specified positions of mutated residues refer to Par j 1 and Par j 2 molecules as identified by SEQ ID NO: and 2, respectively. However, the hybrid protein according to the invention may contain sequences from naturally-occurring molecules of the same class as Par j 1 and Par j 2, such as Par j 1.0102 (Uniprot 004404), Par j 1.0201 (Uniprot Q40905), Par j 1.0101 (Uniprot P43217), Par j 2.0101 (Uniprot P55958), and Par j 2.0101 (Uniprot P55958), which are mutated in Lys residues matching the Lys residues at the above identified positions of SEQ ID NO:1 or 2, in sequence alignments between said natural molecules and SEQ ID NO: or 2.

Par j 1 and Par j 2 proteins may be arranged in any order within the hybrid molecule, and with a "head-to-tail orientation", meaning with this that when the hybrid protein amino-terminal coincides with the amino-terminal of Par j 1 or Par j 2, the hybrid protein carboxy-terminal coincides with the carboxy-terminal of Par j 2 and Par j 1, respectively. According to a preferred embodiment, the hybrid protein amino-terminal is occupied by Par j 1 and the carboxy-terminal by Par j 2.

The linker separating the mutated sequences of Par j 1 and Par j 2 consists preferably of a chain of 8 amino acids, more preferably a chain of two amino acids, even more preferably the GlySer dipeptide.

The above identified Lys residues are preferably replaced by amino acids selected from Ala, Gly, Pro, Leu, Ile, Phe, Thr, Ser, Gln, Asn, Glu, and Asp, more preferably from Ala, Gly, Thr, Ser, Gln, Asn, Glu, and Asp.

In a particularly preferred embodiment of the invention, the hybrid protein has the sequence identified in SEQ ID N. 3.

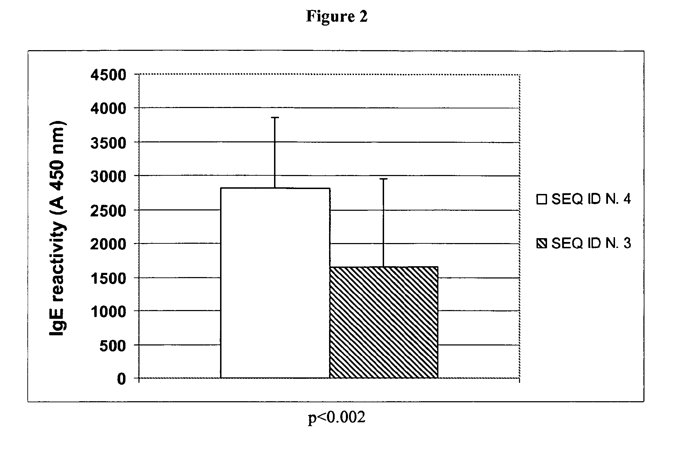

The reactivity of this hybrid protein towards IgEs has been analyzed in twelve sera from Parietaria pollen-allergic subjects by ELISA assay (Fig. 2). When incubated with the sera, the hybrid protein exhibited a mean decrease in the reactivity towards IgEs of 41% compared to wt hybrid (SEQ ID N. 4), with values comprised between 10 and 100%.

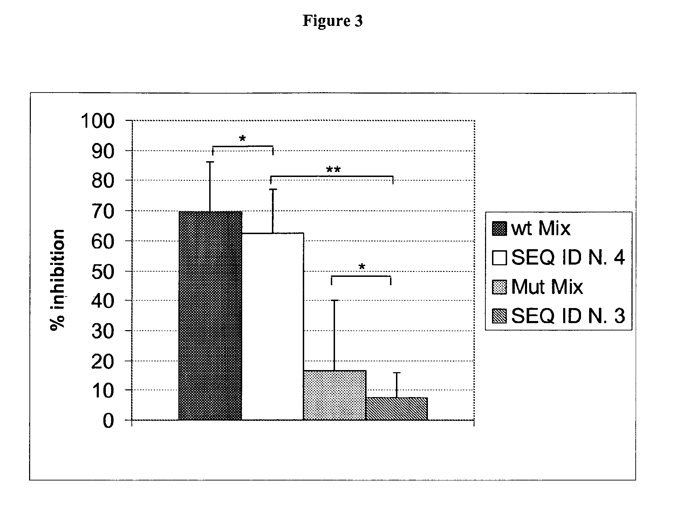

These results have been confirmed by ELISA inhibition experiments, allowing the evaluation of reactivity of homologous epitopes in different proteins. At equimolar (0.150 nM) inhibitor concentrations, the binding between *Parietaria judaica* extract adsorbed on wells and specific IgEs contained in the sera of 14 patients was inhibited, on average, by 69.8% when serum was pre-treated with the mix of single wt allergens, by 16.5% when pre-incubated with the mix of the two mutagenized components, by 62.4% when pre-treated with SEQ ID N. 4, and by 7.6% when pre-incubated with SEQ ID N. 3 (Fig. 3).

Thus, the ability of the mutant hybrid according to the invention to inhibit Parietaria extract binding to specific IgEs is significantly lower than both the wt SEQ ID N. 4 hybrid (p<0.001) and the two mutagenized allergens mixture (p<0.05).

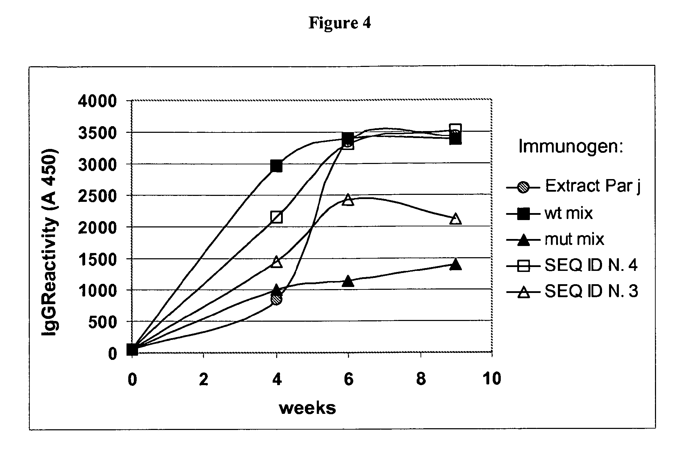

Moreover, the wild-type (SEQ ID N. 4) and hypoallergenic (SEQ ID NO:3) hybrid proteins, when used to immunize Balb/c mice, proved able to induce a specific IgG response to *Parietaria judaica* extract (Fig. 4). Hybrid molecules SEQ ID N. 3 and SEQ ID N. 4 induced an earlier IgG response to Parietaria extract compared to the whole extract. After four weeks from first immunization, corresponding to a week after second immunization, the IgG response was 2.5-fold higher with SEQ ID N. 4, and 1.7-fold higher with SEQ ID N. 3 than that obtained by immunizing with Parietaria extract. Moreover, the IgG antibody response obtained with SEQ ID N. 3 was much stronger than that obtained by immunizing with equimolar amounts of the two single mutagenized variants (mut mix). Conversely, antibodies in the serum of animals immunized with an unrelated antigen were not able to recognize Parietaria extract.

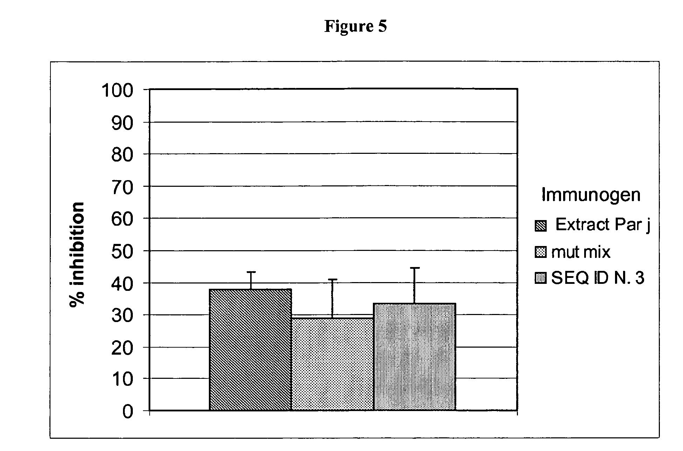

Concerning the induction of protective antibodies capable of blocking the allergen binding to IgEs, it was observed that IgG antibodies produced against SEQ ID N. 3 inhibited the binding of Parietaria allergic-patients' IgEs to Par j total extract (Fig. 5). ELISA inhibition experiments showed that IgGs from mice immunized with SEQ ID N. 3 inhibited IgE reactivity of eleven sera by 33.4% on average (with values from 19.7 to 50.7%), and that antibodies produced against Parietaria total extract inhibited this binding by 37.8% (27.5-47.3%), whereas IgGs obtained by immunizing with the mix of the two mutagenized Par j 1 and Par j 2 allergens (mut mix) inhibited IgE-extract binding, on average, by 28.6%, with values from 13% to 50%.

The hybrid protein according to the invention can be easily prepared by mutagenesis of Par j 1 (SEQ ID NO:5), Par j 2 (SEQ ID NO:6), isoform or natural variants thereof, or of the wt hybrid (SEQ ID N.7) cDNA sequence, by methods known to those skilled in the art (see e.g. 13,18,19).

In a further aspect, therefore, the invention provides a nucleic acid molecule coding for a hybrid protein as herein defined.

In a preferred embodiment, such nucleic acid molecule codes for SEQ ID NO:3 protein and consists of SEQ ID NO:8 sequence.

In a yet further aspect, the invention provides an expression vector containing a nucleic acid molecule as herein defined together with elements for expression control in eukaryotic or prokaryotic cells, such as transcriptions promoters, enhancers, signal sequences and other sequences for transcriptional regulation. The vector can be a plasmid, virus, or any other vector commonly used in genetic engineering.

The invention further comprises a eukaryotic or prokaryotic host cell transformed or transfected with the vector of the invention. Prokaryotic cells such as *Escherichia coli* and *Bacillus subtilis*, or eukaryotic cells such as *Saccharomyces cerevisiae* are generally used for vector cloning and cDNA expression.

Moreover, hypoallergenic hybrid proteins according to the invention can be produced as fusion proteins.

Given the reduced reactivity to IgEs, the hybrid molecule according to the present invention can be suitably used for the preparation of pharmaceutical compositions to be used in immunotherapy of allergic subjects to *Parietaria judaica* pollen.

A further aspect of the invention therefore concerns a pharmaceutical composition comprising an effective amount of a hybrid molecule as provided herein, optionally in combination with other *Parietaria judaica* allergens and with pharmaceutically acceptable vehicles, excipients, and adjuvants. In a preferred embodiment, such pharmaceutical composition is in the form of a suitable vaccine for preventive or therapeutic treatment of allergic diseases, such as bronchial asthma, allergic rhinitis and allergic conjunctivitis. Principles and methods for vaccination are known to those skilled in the art, reference is made to (20) e (21).

The following examples illustrate the invention in more detail.

### EXAMPLES

Unless otherwise indicated, the methods used in the following examples are described in Sambrook, Fritsch ET Maniatis "Molecular Cloning: A Laboratory Manual" II Ed. Vol. 1-2-3 CSH Lab Press 1989.

### EXAMPLE 1 - Construction of a plasmid coding for wild-type Par j 1-Par j 2 hybrid molecule (wtHybrid)

The hybrid molecule containing the genetic information for wild-type Par j 1-Par j 2 hybrid was obtained by fusion of cDNAs coding for the single allergens.

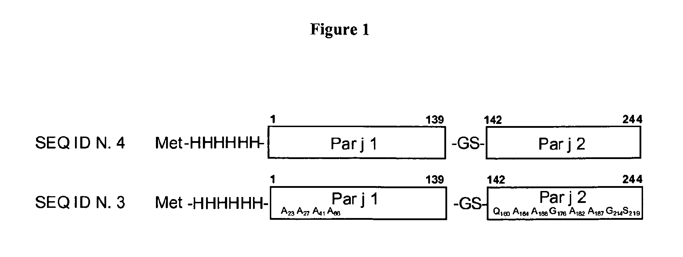

The cDNAs encoding mature Par j 1 and Par j 2 proteins were obtained separately by PCR using **Pj 1 DIM FW** (gcg cga aga aac ctg cgg gac tgt agt g) and **Pj1 DIM RV** (gcg gga tcc ggc ttt ttc cgg tgc ggg ggg) oligonucleotide primers for Par j 1, and **Pj2 FW** (cgc gga tcc gag gag gct tgc ggg) and **PJ2 RV** (gcg gga tcc cta ata gta acc tct gaa aat agt act ttg) oligonucleotide primers for Par j 2. Par j 1 SEQ ID NO:5 and Par j 2 SEQ ID NO:6 clones were used as templates. The amplification product obtained from Par j 1 was re-amplified by replacing **Pj 1 DIM FW** primer with **Pj1 His FW** (gcg cat atg cat cac cat cac cat cac gaa gaa acc tgc ggg act g), whereby a sequence coding for six histidines upstream of Par j 1 sequence was inserted. An Nde I site (containing the ATG) was inserted at 5' of Par j 1 amplification product, and a Bam H I site was inserted at its 3' in place of the stop codon. A Bam H I site was inserted in place of the ATG at 5' of Par j 2, and a Bam H I site was inserted at its 3' after the stop codon. The amplified products were purified, digested with Nde I and Bam H I restriction enzymes (Par j 1), or Bam H I (Par j 2) (restriction sites are underlined in the primers), and subsequently inserted into Nde I/Bam H I sites of pEt 3c vector (Stratagene, La Jolla, CA) to obtain a construct capable of expressing a Par j 1-Par j 2 fusion protein preceded by a sequence of six histidines. Introduction of Bam H I restriction site, that is necessary for cloning of fragments, allowed for insertion of two amino acids (glycine and serine) at the junction of the two proteins without altering the reading frame (Fig. 1).

Clones obtained from single bacterial colonies were sequenced by the Sanger method to verify that base change was correct, and the absence of unspecific mutations in the cDNA.

### EXAMPLE 2 - Construction of a plasmid coding for mutant Par j 1-Par j 2 hybrid molecule (MutHybrid)

The hybrid molecule coding for mutant Par j 1-Par j 2 hybrid was obtained following the method described in EXAMPLE 1 for the wild-type hybrid variant.

The oligonucleotide pairs used in the PCR reaction were identical, while the cDNAs used as templates encoded for two hypoallergenic variants whose sequences are herein identified as SEQ ID NO: 9 (for Par j 1 mutant) and SEQ ID NO: 10 (Par j 2 mutant).

Clones obtained from single bacterial colonies were sequenced by the Sanger method to verify that base change was correct, and the absence of unspecific mutations in the cDNA.

### EXAMPLE 3 - Production of Par j 1 and Par j 2 proteins, respective mutants, wtHybrid and MutHybrid

Wild-type Par j 1 (SEQ ID NO:5) and Par j 2 (SEQ ID NO:6) cDNAs, mutagenized cDNAs (SEQ ID NO: 9 and 10), and engineered wt and Mut Hybrid cDNAs (SEQ ID NO: 7 and 8), preceded by the sequence coding for six histidines, were cloned in an expression vector and expressed in *Escherichia coli* cells according to standard protocols (22,23). Cells were collected by centrifugation and resuspended in 50 mM NaH₂PO₄, 300 mM NaCl buffer, pH 8. Recombinant proteins were isolated after lysis of bacterial cells by sonication and removal of cell particulates by centrifugation. Proteins were purified from the supernatant by affinity chromatography using agarose columns to which nitrilotriacetic acid chelating nickel ions that interact with the six-histidine portion fused to allergen was bound.

### EXAMPLE 4 - Characteristics of sera from allergic subjects

Sera were collected from subjects with a clinical history of seasonal allergy to *Parietaria judaica* pollen, and 3+ and 4+ RAST reactivity specific for Par j 1 and Par j 2 allergens. A pool of sera from non-allergic subjects was used as a negative control (data not shown).

### EXAMPLE 5 - SEQ ID N: 3 hybrid protein reactivity to IgEs of sera from Parietaria allergic patients by ELISA assay

Equal amounts (0.075 nM) of SEQ ID NO: 4 allergen and its mutagenized SEQ ID NO: 3 variant, in 50 mM carbonate/bicarbonate buffer, pH 9.6, were adsorbed onto wells of polystyrene plates for ELISA assay by incubating at 4°C for 16 hours. Wells were then washed with washing solution (60 mM phosphate buffer, pH 6,5, containing 0.05% Tween-20), and free sites were blocked with diluting solution (15% goat serum, 1 mM EDTA, 0.05% Tween-20, in 150 mM phosphate buffer, pH 7.4). Equal aliquots (100 µl) of 12 human positive sera to Par j 1 and Par j 2, or sera from non-allergic subjects (data not shown), at a 1:10 dilution in diluting buffer were added to each sample and incubated at 25°C for 3 hours. After three washes, anti-human IgE peroxidase-conjugated serum diluted 1:3500 in diluting buffer was added, and incubated at 25°C for 1.5 hours. After three washes, colorimetric reaction development was obtained by adding 100 µl TMB reagent (BioFX Laboratories, Owings Mills, MD), and incubating for 20 minutes at 25°C. The reaction was stopped by adding 100 µl 1 N HCl, and evaluated by reading at 450 nm with a spectrophotometer.

### EXAMPLE 6 - ELISA inhibition assay - SEQ ID NO: 4 and SEQ ID NO: 3 inhibit binding of Parietaria judaica extract to IgEs in serum

Equal amounts (1 µg) of *Parietaria judaica* extract in 50 mM carbonate/bicarbonate buffer, pH 9.6, were adsorbed onto wells of polystyrene plates for ELISA assay by incubating at 4°C for 16 hours. Wells were then washed with washing solution (60 mM phosphate buffer, pH 6,5, containing 0.05% Tween-20), and free sites were blocked with diluting solution (15% goat serum, 1 mM EDTA, 0.05% Tween-20, in 150 mM phosphate buffer, pH 7.4). Aliquots (100 µl) of a 1:5 dilution of human RAST 4+ and 3+ sera to Par j 1 and Par j 2 were pre-incubated with equimolar amounts (0.150 nM) of wt, mutagenized or engineered (hybrid) allergen at 25°C for 2 hours. The mixes were then added to each well, and incubated at 4°C for 16 hours. After three washes with 0.06 M phosphate, pH 6.5, 0.05% Tween-20 buffer, anti-human IgE peroxidase-conjugated serum diluted 1:3000 in diluting buffer was added, and incubated at 25°C for 1.5 hours. After three washes, colorimetric reaction development was obtained by adding 100 µl TMB reagent (BioFX Laboratories, Owings Mills, MD), and incubating for 20 minutes at 25°C. The reaction was stopped by adding 100 µl 1 N HCl, and evaluated by reading at 450 nm with a spectrophotometer. Inhibition percentage was calculated by using the following formula: 100 x [(A-B)/A], where A is absorbance at 450 nm in the absence of inhibitor, and B is absorbance in the presence of inhibitor.

**Table. Hybrid wild-type- and mutagenized-molecules inhibit Parietaria judaica - IgE binding**

| serum | **wt Mix** | **Mut Mix** | **SEQ ID NO: 4** | **SEQ ID NO: 3** |
|---|---|---|---|---|
| **1** | 84.2 | 7.0 | 87.6 | 0.4 |
| **2** | 83.4 | 10.1 | 77.7 | -0.6 |
| **3** | 61.2 | 24.0 | 59.7 | -4.2 |
| **4** | 71.8 | 5.4 | 47.7 | 1.0 |
| **5** | 62.7 | 13.9 | 61.5 | 12.9 |
| **6** | 39.9 | 5.8 | 30.6 | 12.5 |
| **7** | 78.9 | 13.6 | 79.3 | 12.6 |
| **8** | 85.4 | 21.8 | 83.9 | 14.8 |
| **9** | 73.7 | 1.7 | 46.5 | 8.9 |
| **10** | 83.4 | 30.9 | 81.6 | 17.3 |
| **11** | 70.7 | 30.8 | 70.5 | 22.5 |
| **12** | 31.0 | 1.5 | 4.0 | -3.4 |
| **13** | 79.6 | 9.6 | 63.4 | 5.5 |
| **14** | 71.6 | 54.8 | 80.0 | 6.7 |
| mean %inhibition | **69.8** | **16.5** | **62.4** | **7.6** |
| standard deviation | 16.52 | 14.77 | 23.63 | 8.20 |

### EXAMPLE 7 - Immunization of Balb/c mice

Five groups of mice each composed of three Balb/c strain female animals (Charles River) were subcutaneously immunized with 200 µl emulsion made of 100 µl complete Freund's adjuvant and equimolar amounts (0.150 nM) of recombinant antigen or *Parietaria judaica* extract (20 µg) in 100 µl saline. Other two boosts were performed after 21 and 42 days by replacing complete adjuvant with incomplete adjuvant. As a control, three mice received the same treatment with an unrelated antigen (data not shown). Four, six, and nine weeks following first immunization, blood collection was performed from jugular vein of mice, and antibody response to the respective immunogen was checked by ELISA.

### EXAMPLE 8 - Analysis of specific IgG response in immunized mice by ELISA assay

Equal amounts (1 µg) of *Parietaria judaica* extract in 50 mM carbonate/bicarbonate buffer, pH 9.6, were adsorbed onto wells of polystyrene plates for ELISA assay by incubating at 4°C for 16 hours. Wells were then washed with washing solution (60 mM phosphate buffer, pH 6,5, containing 0.05% Tween-20), and free sites were blocked with diluting solution (15% goat serum, 1 mM EDTA, 0.05% Tween-20, in 150 mM phosphate buffer, pH 7.4). Sera from mice were pooled based on immunogenic type and time elapsed from first immunization. Equal aliquots (100 µl) of each pool were added to each well at a 1:27000 dilution in diluting buffer, and incubated at 25°C for 3 hours. After three washes, anti-total mouse IgG peroxidase-conjugated serum diluted 1:8000 in buffer diluting was added and incubated at 25°C for 1.5 hours. After three washes, colorimetric reaction development was obtained by adding 100 µl TMB reagent (BioFX Laboratories, Owings Mills, MD), and incubating for 20 minutes at 25°C. The reaction was stopped by adding 100 µl 1 N HCl and read at 450 nm with a spectrophotometer.

### EXAMPLE 9 - ELISA inhibition assay: IgGs against SEQ ID NO: 4 and SEQ ID NO: 3 inhibit the binding between Parietaria judaica extract and IgEs in the sera of Parietaria allergic patients

Equal amounts (1 µg) of *Parietaria judaica* extract in 50 mM carbonate/bicarbonate buffer, pH 9.6, were adsorbed onto wells of polystyrene plates for ELISA assay by incubating at 4°C for 16 hours. Wells were then washed with washing solution (60 mM phosphate buffer, pH 6,5, containing 0.05% Tween-20), and free sites were blocked with diluting solution (15% goat serum, 1 mM EDTA, 0.05% Tween-20, in 150 mM phosphate buffer, pH 7.4). Aliquots (100 µl) of 1:25 diluted pools from mouse sera collected after seven weeks from first immunization were incubated at 4°C for 16 hours. After three washes with 0.06 M phosphate buffer, pH 6.5, 0.05% Tween-20, twelve 1:10 diluted, RAST 4+ and 3+, human sera to Par j 1 and Par j 2 were added at 25°C for 2 hours. After three washes with 0.06 M phosphate buffer, pH 6.5, 0.05% Tween-20, anti-human IgE peroxidase-conjugated serum diluted 1:3500 in diluting buffer was added, and incubated at 25°C for 1.5 hours. After three washes, colorimetric reaction development was obtained by adding 100 µl TMB reagent (BioFX Laboratories, Owings Mills, MD), and incubating for 20 minutes at 25°C. The reaction was stopped by adding 100 µl 1 N HCl and read at 450 nm with a spectrophotometer. Inhibition percentage was calculated by using the following formula: 100 x [(A-B)/A], where A is absorbance at 450 nm in the absence of inhibiting human serum, and B is absorbance in the presence of inhibiting human serum.

### EXAMPLE 10 - Statistical analysis

In the figures, results are expressed as mean values plus corresponding standard deviations.

UNISTAT 5.5 Light for Excel software was used for statistical analyses. Data were analyzed by paired t-Test.

### BIBLIOGRAPHY

1) Vrtala S, (2008) "From allergen genes to new forms of allergy diagnosis and treatment". Allergy; 63(3):299-309.
2) Malling H. J., (1998) "Immunotherapy as an effective tool in allergy treatment". Allergy; 53: 461.
3) Toubi E., Kessel A., Blant A., Golan T.D., (1999) "Follow-up after systemic adverse reactions of immunotherapy". Allergy; 54(6): 617-620.
4) Visco V, Dolecek C, Denépoux S, Le Mao J, Guret C, Rousset F, Guinnepain MT, Kraft D, Valenta R, Weyer A, Banchereau J, Lebecque S. (1996). "Human IgG monoclonal antibodies that modulate the binding of specific IgE to birch pollen Bet 1". J. Immunol. 157: 956-962.
5) Vrtala S, Ball T, Spitzauer s, Pandjaitan B, Suphioglu C, Knox B, Sperr WR, Valent P, Kraft D, Valenta R. (1998). "Immunization with purified natural and recombinant allergens induces mouse IgG1 antibodies that recognize similar epitopes as human IgE and inhibit the human IgE-allergen interaction and allergen-induced basophil degranulation". J Immunol 160: 6137.
6) Singh MB, de Weerd N, Bhalla PL, (1999) "Genetically engineered plant allergens with reduced anaphylactic activity". Int. Arch. Allergy Immmunol.; 119: 75-85.
7) Colombo P, Duro G, Costa MA, Izzo V, Mirisola MG, Locorotondo G, Cocchiara R, Geraci D. (1998). "Parietaria pollen allergens". Allergy; 53:917-921.
8) Costa M.A., Colombo P., Izzo V., Kennedy D., Venturella S., Cocchiara R., Mistrello G., Falagiani P., Geraci D., (1994). "cDNA cloning, expression and primary structure of Par j I, a major allergen of Parietaria judaica pollen". FEBS Lett.; 341:182-186.
9) Duro G., Colombo P., Costa M.A., Izzo V., Porcasi R., Di Fiore R., Locorotondo G., Mirisola M.G., Cocchiara R., Geraci D., (1996). "cDNA cloning, sequence analysis and allergological characterization of Par j 2.0101, a new major allergen of the Parietaria judaica pollen". FEBS Lett.; 399: 295-298.
10) Colombo P., Kennedy D., Ramsdale T., Costa M.A., Duro G., Izzo V., Salvadori S., Guerrini R., Cocchiara R., Mirisola M.G., Wood S., Geraci D., (1998). "Identification of an immunodominant IgE epitope of the Parietaria judaica major allergen". J. Immunol; 160: 2780-2785.
11) Asturias JA, Gomez-Bayon N, Eseverry JL, Martinez A. (2003). "Par j 1 and Par j 2, the major allergens from Parietaria judaica pollen, have similar immunoglobulin E epitopes". Clin Exp Allergy; 33:518-524.
12) Han GW, Lee JY, Song HK et al. (2001). Structural basis of non-specific lipid binding in maize, lipid transfer protein complexes revealed by high-resolution X-ray crystallography". J Mol Biol; 308:263-278.
13) Bonura A, Amoroso S, Locorotondo G, Di Felice G, Tinghino R, Geraci D, Colombo P. (2001) "Hypoallergenic variants of the Parietaria judaica major allergen Par j 1: a member of the non-specific lipid transfer protein plant family". Int Arch Allergy Immunol; 126:32-40.
14) Sturaro M, Viotti A, Genga A, Falagiani P, Mistrello G, Roncarolo D, Zanotta S. "Variants of the major allergen Par j 2 of *Parietaria judaica*". WO 02/22674.
15) Mistrello G, Zanotta S, Roncarolo D, Falagiani P, Viotti A. "Hypoallergenic proteins derived from the major allergen of *Parietaria judaica*".. EP1712560.
16) Gonzalez-Rioja R, Ibarrola I, Arilla C, Ferrer A, Amparo M, Andreu C, Martinez A, Asturias J. (2007). "Genetically engineered hybrid proteins from Parietaria judaica pollen for allergen-specific immunotherapy". J allergy Clin Immunol; 120:602-609, and WO2007/116316.
17) Costa MA, Geraci D, Colombo P, Passantino R, Bonura A. "Hypoallergenic variants of the *Parietaria judaica* major allergens, uses thereof and compositions comprising them". WO 2004/104047 A1.
18) Bonura A, Corinti S, Artale A, Di Felice G, Amoroso S, Melis M, Geraci D, Colombo P. (2007). "A hybrid expressing genetically engineered major allergens of the Parietaria pollen as a tool for specific allergy vaccination". Int Arch Allergy Immunol; 142:274-284.
19) Wang W., Malcolm BA. (2002). "Two-stage polymerase chain reaction protocol allowing introduction of multiple mutations, deletions, and insertions, using QuikChange site-directed mutagenesis". Methods Mol Biol.;182: 37-43.
20) Paul, (1989), "Fundamental Immunology", Raven press, New York.
21) Cryz, S.J. (1991), "Immunotherapy and Vaccines", VCH Verlagsgesellschaft.
22) Younghee Kim. (2004). "Cloning and Expression of a Lipase Gene from Rice (Oryza sativa cv. Dongjin)". Mol. Cells, 18 (1): 40-45.
23) Asturias JA, Ibarrola I, Eseverri JL, Arilla MC, Gonzales-Rioja R, Martinez A. (2004). "PCR-based cloning and immunological characterization of Parietaria judaica pollen profilin". J Investig Allergol Clin Immunol, 14: 43-48.

## Claims

1. Hybrid protein consisting of mutated Par j 1 and Par j 2 allergens, optionally separated by a linker, wherein:
a) Par j 1 sequence is either SEQ ID NO:1 or a sequence of a naturally-occurring protein identical to SEQ ID NO:1 by at least 95%,
and
in said Par j 1 sequence, the Lys residues at positions 23, 27, 41 and 66 of SEQ ID NO:1, or at the corresponding positions of said naturally-occurring protein wherein matching Lys residues are present in respective sequence alignments, are replaced with neutral, polar or acidic amino acids;
b) Par j 2 sequence is either SEQ ID NO:2 or a sequence of a naturally-occurring protein identical to SEQ ID NO:2 by at least 95%,
and
in said Par j 2 sequence, the Lys residues at positions 19, 23, 27, 35, 41, 46, 73 and 78 of SEQ ID NO:2, or at the corresponding positions of said naturally-occurring protein wherein matching Lys residues are present in respective sequence alignments, are replaced with neutral, polar or acidic amino acids;
c) Par j 1 and Par j 2 sequences, as defined above, can be indifferently located at the N- or C-terminals of the hybrid protein, provided they have head-to-tail orientation;
d) if present, said linker consists of 1 to 8 amino acids.

2. Hybrid protein according to claim 1, wherein said Lys residues are substituted with amino acids selected from Ala, Gly, Pro, Leu, Ile, Phe, Thr, Ser, Gln, Asn, Glu, Asp.

3. Hybrid protein according to claim 2, wherein said amino acids are selected from Ala, Gly, Thr, Ser, Gln, Asn, Glu, Asp.

4. Hybrid protein according to claim 1, wherein said linker is a dipeptide.

5. Hybrid protein according to claim 1, wherein Par j 1 and Par j 2 are located at the N- and C-terminals, respectively.

6. Hybrid protein according to claim 1, having sequence SEQ ID NO:3.

7. Nucleic acid molecule coding for a hybrid protein according to claims 1-6.

8. Nucleic acid molecule according to claim 6, having sequence SEQ ID NO: 8.

9. Expression vector containing the nucleic acid molecule of claims 7-8.

10. Host cell containing the vector of claim 9.

11. Pharmaceutical composition comprising an effective amount of a hybrid protein according to claims 1-6 together with pharmaceutically acceptable carriers and excipients.

12. Pharmaceutical composition according to claim 11, which is in the form of a vaccine suitable for immunotherapy of allergic diseases.

13. Hybrid protein according to claims 1-6, or pharmaceutical composition according to claims 11-12, for the preventive or therapeutic treatment of allergic diseases caused by *Parietaria judaica* pollen.

14. Hybrid protein according to claims 1-6, or pharmaceutical composition according to claims 11-12, for the treatment of allergic bronchial asthma or allergic rhinoconjunctivitis.

## Patentansprüche

1. Hybridprotein, bestehend aus mutierten Par-j-1- und Par-j-2-Allergenen, die gegebenenfalls durch einen Linker voneinander betrennt sind, wobei:
a) es sich bei der Par-j-1-Sequenz entweder um SEQ ID Nr. 1 oder eine Sequenz eines natürlich vorkommenden Proteins, das zu wenigstens 95% mit SEQ ID Nr. 1 identisch ist, handelt;
und
in der Par-j-1-Sequenz die Lys-Reste auf den Positionen 23, 27, 41 und 66 von SEQ ID Nr. 1 oder auf den entsprechenden Positionen des natürlich vorkommenden Proteins, wobei in den jeweiligen Sequenzalignments übereinstimmende Lys-Reste vorhanden sind, durch neutrale, polare oder saure Aminosäuren ersetzt sind;
b) es sich bei der Par-j-2-Sequenz entweder um SEQ ID Nr. 2 oder eine Sequenz eines natürlich vorkommenden Proteins, das zu wenigstens 95% mit (SEQ ID Nr. 2 identisch ist, handelt;
und
in der Par-j-2-Sequenz die Lys-Reste auf den Positionen 19, 23, 27, 35, 41, 46, 73 und 78 von SEQ ID Nr. 2 oder auf den entsprechenden Positionen des natürlich vorkommenden Proteins, wobei in den jeweiligen Sequenzalignments übereinstimmende Lys-Reste vorhanden sind, durch neutrale, polare oder saure Aminosäuren ersetzt sind;
c) die oben definierten Par-j-1- und Par-j-2-Sequenzen sich genauso gut am N- wie am C-Terminus des Hybridproteins befinden können, vorausgesetzt, dass sie Kopf-Schwanz-Orientierung aufweisen;
d) der Linker, falls vorhanden, aus 1 bis 8 Aminosäuren besteht.

2. Hybridprotein gemäß Anspruch 1, wobei die Lys-Reste durch Aminosäuren, die aus Ala, Gly, Pro, Neu, Ile, Phe, Thr, Ser, Gln, Asn, Glu, Asp ausgewählt sind, substituiert sind.

3. Hybridprotein gemäß Anspruch 2, wobei die Aminosäuren aus Ala, Gly, Thr, Ser, Gln, Asn, Glu, Asp ausgewählt sind.

4. Hybridprotein gemäß Anspruch 1, wobei der Linker ein Dipeptid ist.

5. Hybridprotein gemäß Anspruch 1, wobei sich Par-j-1 und Par-j-2 jeweils am N-Terminus und am C-Terminus befinden.

6. Hybridprotein gemäß Anspruch 1, das die Sequenz SEQ ID Nr. 3 aufweist.

7. Nucleinsäuremolekül, das für ein Hybridprotein gemäß den Ansprüchen 1 bis 6 codiert.

8. Nucleinsäuremolekül gemäß Anspruch 6, das die Sequenz SEQ ID Nr. 8 aufweist.

9. Expressionsvektor, der das Nucleinsäuremolekül gemäß Anspruch 7-8 enthält.

10. Wirtszelle, die den Vektor gemäß Anspruch 9 enthält.

11. Pharmazeutische Zusammensetzung, die eine wirksame Menge eines Hybridproteins gemäß den Ansprüchen 1 bis 6 zusammen mit pharmazeutisch annehmbaren Trägern und Arzneimittelhilfsstoffen umfasst.

12. Pharmazeutische Zusammensetzung gemäß Anspruch 11, die in Form eines Impfstoffs vorliegt, der für die Immuntherapie von allergischen Krankheiten geeignet ist.

13. Hybridprotein gemäß den Ansprüchen 1 bis 6 oder pharmazeutische Zusammensetzung gemäß den Ansprüchen 11 bis 12 zur präventiven oder therapeutischen Behandlung von allergischen Krankheiten, die durch Pa*rietaria-judaica-Pollen* verursacht sind.

14. Hybridprotein gemäß den Ansprüchen 1 bis 6 oder pharmazeutische Zusammensetzung gemäß den Ansprüchen 11 bis 12 zur Behandlung von allergischem Bronchialasthma oder allergischer Rhinokonjunktivitis.

## Revendications

1. Protéine hybride consistant en 2 allergènes Par j 1 et Par j 2 mutés, éventuellement séparés par un lieur, dans laquelle :
a) la séquence de Par j 1 est soit SEQ ID NO: 1 soit une séquence d'une protéine présente naturellement et à 95% au moins identique à SEQ ID NO: 1.
et
dans ladite séquence de Par j 1, les résidus Lys aux positions 23, 27, 41 et 66 de SEQ ID NO: 1, ou aux positions correspondantes de ladite protéine présente naturellement dans laquelle les résidus Lys correspondants sont présents dans les alignements respectifs des séquences, sont remplacés par des acides aminés neutres, polaires ou acides ;
b) la séquence de Par j 2 est soit SEQ ID NO: 2 soit une séquence d'une protéine présente naturellement et à 95% au moins identique à SEQ ID NO: 2.
et
dans ladite séquence de Par j 2, les résidus Lys aux positions 19, 23, 27, 35, 41, 46, 73 et 78 de SEQ ID NO: 2, ou aux positions correspondantes de ladite protéine présente naturellement dans laquelle les résidus Lys correspondants sont présents dans les alignements des séquences respectives, sont remplacés par des acides aminés neutres, polaires ou acides ;
c) les séquences de Par j 1 et Par j 2, telles que définies ci-dessus, peuvent être situées indifféremment aux extrémités N- ou C-terminales de la protéine hybride, à condition qu'elles aient l'orientation tête-à-queue ;
d) s'il est présent, ledit lieur se compose de 1 à 8 acides aminés.

2. Protéine hybride selon la revendication 1, dans laquelle lesdits résidus Lys sont remplacés par des acides aminés choisis parmi Ala, Gly, Pro, Leu, Ile, Phe, Thr, Ser, Gln, Asn, Glu, Asp.

3. Protéine hybride selon la revendication 2, dans laquelle lesdits acides aminés sont choisis parmi Ala, Gly, Thr, Ser, Gln, Asn, Glu, Asp.

4. Protéine hybride selon la revendication 1, dans laquelle ledit lieur est un dipeptide.

5. Protéine hybride selon la revendication 1, dans laquelle Par j 1 et Par j 2 sont respectivement situés aux extrémités N- et C-terminales.

6. Protéine hybride selon la revendication 1, ayant la séquence de SEQ ID NO: 3.

7. Molécule d'acide nucléique codant pour une protéine hybride selon les revendications 1 à 6.

8. Molécule d'acide nucléique selon la revendication 6, ayant la séquence de SEQ ID NO: 8.

9. Vecteur d'expression contenant la molécule d'acide nucléique selon les revendications 7 à 8.

10. Cellule hôte contenant le vecteur selon la revendication 9.

11. Composition pharmaceutique comprenant une quantité efficace d'une protéine hybride selon les revendications 1 à 6 conjointement à des supports et des excipients pharmaceutiquement acceptables.

12. Composition pharmaceutique selon la revendication 11, qui est sous la forme d'un vaccin approprié pour une immunothérapie des maladies allergiques.

13. Protéine hybride selon les revendications 1 à 6, ou une composition pharmaceutique selon les revendications 11 à 12, destinée au traitement préventif ou thérapeutique des maladies allergiques provoquées par le pollen de *Parietaria judaica.*

14. Protéine hybride selon les revendications 1 à 6, ou une composition pharmaceutique selon les revendications 11 à 12, destinée au traitement de l'asthme bronchique allergique ou de la rhino-conjonctivite allergique.


### SEQUENCE LISTING

<110> LOFARMA S.P.A.
<120> HYBRID PROTEINS FROM PARIETARIA JUDAICA MAJOR ALLERGENS AND USES THEREOF
<130> 8887MEUR
<160> 15
<170> PatentIn version 3.3
<210> 1
   <211> 139
   <212> PRT
   <213> Parietaria judaica
<400> 1
   Glu Glu Thr Cys Gly Thr Val Val Gly Ala Leu Met Pro Cys Leu Pro
   1               5                   10                  15
   Phe Val Gln Gly Lys Glu Lys Glu Pro Ser Lys Gly Cys Cys Ser Gly
               20                  25                  30
   Ala Lys Arg Leu Asp Gly Glu Thr Lys Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Lys Leu Val Ser Glu Val Pro Lys His Cys Gly Ile Val Asp Ser
   65                  70                  75                  80
   Lys Leu Pro Pro Ile Asp Val Asn Met Asp Cys Lys Thr Leu Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala
       130                 135
<210> 2
   <211> 102
   <212> PRT
   <213> Parietaria judaica
<400> 2
   Glu Glu Ala Cys Gly Lys Val Val Gln Asp Ile Met Pro Cys Leu His
   1               5                   10                  15
   Phe Val Lys Gly Glu Glu Lys Glu Pro Ser Lys Glu Cys Cys Ser Gly
               20                  25                  30
   Thr Lys Lys Leu Ser Glu Glu Val Lys Thr Thr Glu Gln Lys Arg Glu
           35                  40                  45
   Ala Cys Lys Cys Ile Val Arg Ala Thr Lys Gly Ile Ser Gly Ile Lys
       50                  55                  60
   Asn Glu Leu Val Ala Glu Val Pro Lys Lys Cys Asp Ile Lys Thr Thr
   65                  70                  75                  80
   Leu Pro Pro Ile Thr Ala Asp Phe Asp Cys Ser Lys Ile Gln Ser Thr
                   85                  90                  95
   Ile Phe Arg Gly Tyr Tyr
               100
<210> 3
   <211> 243
   <212> PRT
   <213> Unknown
<220>
   <223> synthetic sequence
<400> 3
   Glu Glu Thr Cys Gly Thr Val Val Gly Ala Leu Met Pro Cys Leu Pro
   1               5                   10                  15
   Phe Val Gln Gly Lys Glu Ala Glu Pro Ser Ala Gly Cys Cys Ser Gly
               20                  25                  30
   Ala Lys Arg Leu Asp Gly Glu Thr Ala Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Ala Leu Val Ser Glu Val Pro Lys His Cys Gly Ile Val Asp Ser
   65                  70                  75                  80
   Lys Leu Pro Pro Ile Asp Val Asn Met Asp Cys Lys Thr Leu Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala Gly Ser Glu Glu Ala
       130                 135                 140
   Cys Gly Lys Val Val Gln Asp Ile Met Pro Cys Leu His Phe Val Gln
   145                 150                 155                 160
   Gly Glu Glu Ala Glu Pro Ser Ala Glu Cys Cys Ser Gly Thr Lys Gly
                   165                 170                 175
   Leu Ser Glu Glu Val Ala Thr Thr Glu Gln Ala Arg Glu Ala Cys Lys
               180                 185                 190
   Cys Ile Val Arg Ala Thr Lys Gly Ile Ser Gly Ile Lys Asn Glu Leu
           195                 200                 205
   Val Ala Glu Val Pro Gly Lys Cys Asp Ile Ser Thr Thr Leu Pro Pro
       210                 215                 220
   Ile Thr Ala Asp Phe Asp Cys Ser Lys Ile Gln Ser Thr Ile Phe Arg
   225                 230                 235                 240
   Gly Tyr Tyr
<210> 4
   <211> 243
   <212> PRT
   <213> Unknown
<220>
   <223> synthetic sequence
<400> 4
   Glu Glu Thr Cys Gly Thr Val Val Gly Ala Leu Met Pro Cys Leu Pro
   1               5                   10                  15
   Phe Val Gln Gly Lys Glu Lys Glu Pro Ser Lys Gly Cys Cys Ser Gly
               20                  25                  30
   Ala Lys Arg Leu Asp Gly Glu Thr Lys Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Lys Leu Val Ser Glu Val Pro Lys His Cys Gly Ile Val Asp Ser
   65                  70                  75                  80
   Lys Leu Pro Pro Ile Asp Val Asn Met Asp Cys Lys Thr Leu Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala Gly Ser Glu Glu Ala
       130                 135                 140
   Cys Gly Lys Val Val Gln Asp Ile Met Pro Cys Leu His Phe Val Lys
   145                 150                 155                 160
   Gly Glu Glu Lys Glu Pro Ser Lys Glu Cys Cys Ser Gly Thr Lys Lys
                   165                 170                 175
   Leu Ser Glu Glu Val Lys Thr Thr Glu Gln Lys Arg Glu Ala Cys Lys
               180                 185                 190
   Cys Ile Val Arg Ala Thr Lys Gly Ile Ser Gly Ile Lys Asn Glu Leu
           195                 200                 205
   Val Ala Glu Val Pro Lys Lys Cys Asp Ile Lys Thr Thr Leu Pro Pro
       210                 215                 220
   Ile Thr Ala Asp Phe Asp Cys Ser Lys Ile Gln Ser Thr Ile Phe Arg
   225                 230                 235                 240
   Gly Tyr Tyr
<210> 5
   <211> 420
   <212> DNA
   <213> Parietaria judaica
<400> 5
   gaagaaacct gcgggactgt agtgggagcg ctgatgccgt gcctgccgtt cgtgcagggg 60
   aaagagaaag agccgtcaaa ggggtgctgc agcggcgcca aaagattgga cggggagacg 120
   aagacggggc cgcagagggt gcacgcttgt gagtgcatcc agaccgccat gaagacctat 180
   tccgacatcg acgggaaact cgtcagcgag gtccccaagc actgcggcat cgttgacagc 240
   aagctcccgc ccattgacgt caacatggac tgcaagacac ttggagtggt acctcggcaa 300
   ccccaacttc cagtctctct ccgtcatggt cccgtcacgg gcccaagtga ccccgcccac 360
   aaagcacggt tggagagacc ccagattaga gttccgcccc ccgcaccgga aaaagcctaa 420
<210> 6
   <211> 309
   <212> DNA
   <213> Parietaria judaica
<400> 6
   gaggaggctt gcgggaaagt ggtgcaggat ataatgccgt gcctgcattt cgtgaagggg 60
   gaggagaagg agccgtcgaa ggagtgctgc agcggcacga agaagctgag cgaggaggtg 120
   aagacgacgg agcagaagag ggaggcctgc aagtgcatag tgcgcgccac gaagggcatc 180
   tccggtatca aaaatgaact tgtcgccgag gtccccaaga agtgcgatat taagaccact 240
   ctcccgccca tcaccgccga cttcgactgc tccaagatcc aaagtactat tttcagaggt 300
   tactattag 309
<210> 7
   <211> 732
   <212> DNA
   <213> Unknown
<220>
   <223> synthetic sequence
<400> 7
   gaagaaacct gcgggactgt agtgggagcg ctgatgccgt gcctgccgtt cgtgcagggg      60
   aaagagaaag agccgtcaaa ggggtgctgc agcggcgcca aaagattgga cggggagacg     120
   aagacggggc cgcagagggt gcacgcttgt gagtgcatcc agaccgccat gaagacctat     180
   tccgacatcg acgggaaact cgtcagcgag gtccccaagc actgcggcat cgttgacagc     240
   aagctcccgc ccattgacgt caacatggac tgcaagacac ttggagtggt acctcggcaa     300
   ccccaacttc cagtctctct ccgtcatggt cccgtcacgg gcccaagtga ccccgcccac     360
   aaagcacggt tggagagacc ccagattaga gttccgcccc ccgcaccgga aaaagccgga     420
   tccgaggagg cttgcgggaa agtggtgcag gatataatgc cgtgcctgca tttcgtgaag     480
   ggggaggaga aggagccgtc gaaggagtgc tgcagcggca cgaagaagct gagcgaggag     540
   gtgaagacga cggagcagaa gagggaggcc tgcaagtgca tagtgcgcgc cacgaagggc     600
   atctccggta tcaaaaatga acttgtcgcc gaggtcccca agaagtgcga tattaagacc     660
   actctcccgc ccatcaccgc cgacttcgac tgctccaaga tccaaagtac tattttcaga     720
   ggttactatt ag                                                         732
<210> 8
   <211> 732
   <212> DNA
   <213> Unknown
<220>
   <223> synthetic sequence
<400> 8
   gaagaaacct gcgggactgt agtgggagcg ctgatgccgt gcctgccgtt cgtgcagggg     60
   aaagaggcag agccgtcagc ggggtgctgc agcggcgcca aaagattgga cggggagacg    120
   gcgacggggc cgcagagggt gcacgcttgt gagtgcatcc agaccgccat gaagacctat    180
   tccgacatcg acggggcact cgtcagcgag gtccccaagc actgcggcat cgttgacagc    240
   aagctcccgc ccattgacgt caacatggac tgcaagacac ttggagtggt acctcggcaa    300
   ccccaacttc cagtctctct ccgtcatggt cccgtcacgg gcccaagtga ccccgcccac    360
   aaagcacggt tggagagacc ccagattaga gttccgcccc ccgcaccgga aaaagccgga    420
   tccgaggagg cttgcgggaa agtggtgcag gatataatgc cgtgcctgca tttcgtgcag    480
   ggggaggagg cggagccgtc ggcggagtgc tgcagcggca cgaaggggct gagcgaggag    540
   gtggcgacga cggagcaggc gagggaggcc tgcaagtgca tagtgcgcgc cacgaagggc    600
   atctccggta tcaaaaatga acttgtcgcc gaggtccccg ggaagtgcga tattagcacc    660
   actctcccgc ccatcaccgc tgacttcgac tgctccaaga tccaaagtac tattttcaga    720
   ggttactatt ag                                                        732
<210> 9
   <211> 420
   <212> DNA
   <213> Unknown
<220>
   <223> synthetic sequence
<400> 9
   gaagaaacct gcgggactgt agtgggagcg ctgatgccgt gcctgccgtt cgtgcagggg     60
   aaagaggcag agccgtcagc ggggtgctgc agcggcgcca aaagattgga cggggagacg    120
   gcgacggggc cgcagagggt gcacgcttgt gagtgcatcc agaccgccat gaagacctat    180
   tccgacatcg acggggcact cgtcagcgag gtccccaagc actgcggcat cgttgacagc    240
   aagctcccgc ccattgacgt caacatggac tgcaagacac ttggagtggt acctcggcaa    300
   ccccaacttc cagtctctct ccgtcatggt cccgtcacgg gcccaagtga ccccgcccac    360
   aaagcacggt tggagagacc ccagattaga gttccgcccc ccgcaccgga aaaagcctaa    420
<210> 10
   <211> 309
   <212> DNA
   <213> Unknown
<220>
   <223> synthetic sequence
<400> 10
   gaggaggctt gcgggaaagt ggtgcaggat ataatgccgt gcctgcattt cgtgcagggg     60
   gaggaggcgg agccgtcggc ggagtgctgc agcggcacga aggggctgag cgaggaggtg    120
   gcgacgacgg agcaggcgag ggaggcctgc aagtgcatag tgcgcgccac gaagggcatc    180
   tccggtatca aaaatgaact tgtcgccgag gtccccggga agtgcgatat tagcaccact    240
   ctcccgccca tcaccgccga cttcgactgc tccaagatcc aaagtactat tttcagaggt    300
   tactattag                                                            309
<210> 11
   <211> 28
   <212> DNA
   <213> Unknown
<220>
   <223> oligonucleotide primer
<400> 11
   gcgcgaagaa acctgcggga ctgtagtg 28
<210> 12
   <211> 30
   <212> DNA
   <213> Unknown
<220>
   <223> oligonucleotide primer
<400> 12
   gcgggatccg gctttttccg gtgcgggggg 30
<210> 13
   <211> 24
   <212> DNA
   <213> Unknown
<220>
   <223> oligonucleotide primer
<400> 13
   cgcggatccg aggaggcttg cggg 24
<210> 14
   <211> 39
   <212> DNA
   <213> Unknown
<220>
   <223> oligonucleotide primer
<400> 14
   gcgggatccc taatagtaac ctctgaaaat agtactttg 39
<210> 15
   <211> 46
   <212> DNA
   <213> Unknown
<220>
   <223> oligonucleotide primer
<400> 15
   gcgcatatgc atcaccatca ccatcacgaa gaaacctgcg ggactg 46